# EUROPEAN PATENT SPECIFICATION

(11) **EP 2 947 456 B1**
(45) Date of publication and mention of the grant of the patent: **23.10.2019**
(21) Application number: 15168859.5
(22) Date of filing: 22.05.2015
(51) Int. Cl.: G01N 33/38, G01N 27/02

(54) **METHOD FOR POSITIONING A SENSOR FOR CONCRETE MONITORING**
VERFAHREN ZUR POSITIONIERUNG EINES SENSORS ZUR BETONÜBERWACHUNG
PROCÉDÉ POUR POSITIONNER UN CAPTEUR DE SURVEILLANCE DE BÉTON

(30) Priority: 22.05.2014 BE 201400393; 22.05.2014 BE 201400394
(43) Date of publication of application: 25.11.2015
(73) Proprietor: Zensor BVBA, 1040 Brussel (BE)
(72) Inventor: Van Ingelgem, Yves, 3404 Landen (BE); De Wilde, Daan, 1800 Vilvoorde (BE); Nieubourg, Gori, 1050 Halle (BE)
(74) Representative: Gyi, Jeffrey Ivan

(56) References cited:
- CA-A1- 2 322 931
- US-B1- 7 088 115
- Steve Lyon ET AL: "Corrosion Monitoring Systems and Sensors to Track Material Durability in Concrete Structures", , October 2013 (2013-10), XP055155299, Retrieved from the Internet: URL:http://www.intertek.com/uploadedFiles/ Intertek/Divisions/Analytical_Services/Med ia/PDFs/Intertek-CAPCIS%20Corrosion%20Moni toring%20Systems%20and%20Sensors.pdf [retrieved on 2014-11-26]
- CARMEN ANDRADE ET AL: "Embedded sensors for the monitoring of corrosion parameters in concrete structures", NDTCE'09, NON-DESTRUCTIVE TESTING IN CIVIL ENGINEERING, 3 July 2009 (2009-07-03), XP055155302,
- WILLIAM JOHN MCCARTER ET AL: "Sensor systems for use in reinforced concrete structures", CONSTRUCTION AND BUILDING MATERIALS, vol. 18, no. 6, July 2004 (2004-07), pages 351-358, XP055154209, ISSN: 0950-0618, DOI: 10.1016/j.conbuildmat.2004.03.008

## Description

### FIELD OF THE INVENTION

The present invention provides a method for applying an electrode in building aggregates that are settable, i.e. that harden over time, such as grout, preferably used in a mechanical connection. The present specification describes a method for verifying the state of a grout, preferably used as in a mechanical connection.

### BACKGROUND OF THE INVENTION

In the present context, grout is described as product formed by mixing cement, sand, water and possibly additives and fillers. Grout is very well suited for realizing mechanical connections between constructions or parts of constructions.

The use and the properties of grout are similar to those of concrete. Therefore, the invention can be applied as well for monitoring concrete constructions, or in the monitoring of other building aggregates.

Without limiting the invention to these applications, the invention is well suited for application in offshore constructions such as wind turbines, offshore substations, oil rigs, etc. Such offshore constructions are often kept in place through one or more piles that are partially embedded in the seabed. The main construction is fixed to these piles using grout. Other possible applications are foundations of tidal and wave energy turbines, tidal and classical hydropower dams, bridges, tunnels, and water purification plants.

Grout in mechanical connections between a pile and a main construction is subjected to significant forces to which the main construction is subjected and, in case of offshore constructions, the grout is also subjected to river- or seawater. These external influences can result in damaging or degradation of the grout. It is of upmost importance that the degradation is identified as soon as possible such that appropriate measures can be taken as soon as possible. After all, grout damage or degradation can give rise to failure of mechanical connections realized through the grout. This can have disastrous results for offshore constructions.

One option for checking the state of grouted connections is collecting core drill samples from the grouted connection. This is, however, a destructive method, resulting in damage to the grout.

A better method makes use of measuring instruments such as electrodes that are located on specific locations in the grout and between which a measuring signal such as a potential or current signal is applied. Damage to the grout can be derived from the resulting signal.

Positioning the measuring instruments or electrodes in the grout needs to be done with great precision as the accuracy and correctness of the measurements will depend on it.

Positioning the electrodes during the pouring can result in deformation of or damage to the electrodes as the grout is very stiff and has a high viscosity. After pouring, positioning the electrodes in the grout may be very difficult or even impossible. After the grout has set sufficiently, i.e. after the grout has developed load bearing capability, embedding sensors non-destructively is impossible.

Setting of grout is a gradual process which, in some cases, can take many years to complete. Grout, a material typically comprising Portland cement and sand, is typically easily workable when freshly applied. It gradual hardens "sets" over time, typically reaching at least 70% of its final strength after about 21 days.

One of the goals of the present invention is positioning the electrodes in the grout in a good way as to avoid them getting damaged and to make sure they are located on exactly the desired location.

The present specification describes a method for verifying the state of a grout used in a mechanical connection, for example mechanical connections in offshore constructions such as wind turbines, offshore substations, oil rigs, etc... Such offshore constructions are often kept in place through one or more piles that are partially embedded in the seabed. The main construction is fixed to these piles using a grout.

In case of for example wind turbines a so-called transition piece is placed over the pile, or the transition piece is slit into the pile, wherein both cases a grout is applied between pile and transition piece. The grout provides a strong mechanical connection between the pile and the transition piece. This connection is located most often below the water level. The transition piece is part of the wind turbine, or will be connected to it. The grout in the mechanical connection will be subjected to significant forces exercising on the structure and, in case of offshore constructions, also to sea- or river water. These external influences can result in damaging or degradation of the grout. It is of upmost importance that the degradation is identified as soon as possible such that the appropriate measures can be taken as soon as possible: damage or degradation of the grout can give rise to failure of the mechanical connection realized through the grout. This can have disastrous results for the offshore construction.

One option to check the state of the grouted connection is collecting core drill samples from the grout. This is, however, a destructive method, resulting in damage to the grout.

An alternative is using optical fibers in the grout. These are applied during pouring of the grout. These optical fibers can also be used as a sensor to detect deformations or stresses in the grout. An advantage is that the optical fibers are well suited for detecting forces. A disadvantage of optical fibers is that they are very expensive and also very delicate. As a result it proves to be a technical challenge to install the optical fibers without inducing damage to the fibers.

The present invention targets at solving at least one of the aforementioned disadvantages. Steve Lyon ET AL: "Corrosion Monitoring Systems and Sensors to Track Material Durability in Concrete Structures", October 2013, XP055155299 and CARMEN ANDRADE ET AL: "Embedded sensors for the monitoring of corrosion parameters in concrete structures",NDTCE'09, NON-DESTRUCTIVE TESTING IN CIVIL ENGINEERING, 3 July 2009, XP055155302, disclose methods of embedding sensors into fresh concrete.

### SUMMARY OF THE INVENTION

Outside the scope of the invention, the present specification describes a method for monitoring the state of grout (9') used in a mechanical connection, wherein the method uses a first inert electrode (10') located in the grout (9') and either a steel component (4', 6') which is part of the mechanical connection or which is located in its vicinity, or a second inert electrode (10') which is located in the grout (9'), wherein the inert electrodes (10') and the steel component (4', 6') are electrically connectable, thereby forming a circuit (12'), wherein the method comprises the following steps:
- applying a varying current or potential signal in the circuit (12');
- respectively measuring the resulting potential or current signal in the circuit (12').

In some embodiments, the present invention comprises a method for applying an electrode (7) in grout (6) used in a mechanical connection, wherein the electrode (7) is used for determining the condition of the grout (6), comprising the following steps:
- embedding the electrode (7) in grout (6) such that the electrode (7) is encased with grout (6), wherein a breakable connection is additionally partially embedded into the grout (6) such that the breakable connection is protruding at least partially from the grout (6);
- connecting the grout- (6) encased electrode (10), using the breakable connection, to a rod (11) at a desired location;
- positioning the rod (11) with the electrode (7) in the desired location in the volume (12) of the mechanical connection where the grout will be applied;
- applying the grout (6) in the mechanical connection, for example through pouring;
- breaking or rupturing the breakable connection by moving or displacing the rod (11) with respect to the electrode (7);
- removing the rod (11) from the grout (6).

In general, the present invention comprises a method according to claim 1 for applying an electrode in a building material that is settable, wherein the electrode is used for determining the condition of the building material, comprising the following steps:
- embedding the electrode in the building material such that the electrode is encased with the building material, thereby forming an encased electrode, wherein a breakable connection is additionally partially embedded into the building material such that the breakable connection at least partially protrudes from the casing of the cased electrode;
- connecting the encased electrode using the breakable connection to a rod;
- positioning the rod with the encased electrode in a desired location in a volume where the building material will be applied;
- applying the building material in the volume, for example through pouring;
- breaking or rupturing the breakable connection by moving or displacing the rod (11) with respect to the electrode;
- removing the rod from the building material.

According to the present specification, the building material that is settable may be a building aggregate.

In some embodiments, the rod (11) is positioned to protrude at least partially above the aforementioned volume when positioning the rod (11), wherein, preferably, the length of the rod (11) exceeds the depth (C) at which the electrode (7) is to be positioned in the building material (6) by at least fifty centimeters.

In some embodiments, at least two magnets (13) are used for applying the rod (11) at a desired location, wherein the magnets (13) are attached to the rod (11), and wherein the magnets (13) are positioned on a steel part (2, 5), for example a steel part of a mechanical connection.

In some embodiments, both magnets (13) are separated from each other by at least 40 centimeters, preferably 10 centimeters.

In some embodiments, the breakable connection is broken or ruptured by rotating the rod (11).

In some embodiments, multiple electrodes (7) are attached to a single rod (11).

In some embodiments, multiple electrodes are encased in the building material during the process of encasing the electrodes (7) with the building material, thereby forming encased electrodes.

Outside the scope of the invention, the present specification also describes a method for monitoring the state of load-bearing building aggregates, wherein the method uses a first inert electrode (10') located in the building aggregate and either a steel component (4', 6') which is inside the load-bearing building aggregate or which is located in its vicinity, or a second inert electrode (10') which is located in load-bearing building aggregate, wherein the inert electrodes (10') and the steel component (4', 6') are electrically connectable, thereby forming a circuit (12'), wherein the method comprises the following steps:
- applying a varying current or potential signal in the circuit (12'); and,
- respectively measuring the resulting potential or current signal in the circuit (12').

According to the present specification, the method for monitoring the state of load-bearing building aggregates described above, and which is outside the scope of the invention, may further comprise the step: determining or registering the amplitude of the measured resulting signal, and may preferably further comprise the step: registering the variation of the amplitude of the resulting signal with time. The imposed signal may have a sinusoidal shape, and the imposed signal may preferably have a constant amplitude.

The method for monitoring the state of load-bearing building aggregates outside the scope of the invention may further comprise the steps:
- providing multiple inert electrodes located in building aggregate;
- electrically coupling the multiple inert electrodes with a steel component and/or with another inert electrode thereby forming different circuits;
- applying a varying signal, e.g. a voltage, to the circuits;
- measuring a resulting signal, e.g. a current, in the circuits;
- extracting the location of building aggregate damage from the resulting signals measured in the circuits.

The inert electrodes (10') may be made of titanium and/or the inert electrodes (10') may be coated with a mixed metal oxide or platinum.

A logical unit (11') may be used that is electrically connected to the inert electrodes (10') and to the steel component (4',6'), wherein the logical unit (11') realizes the electrical connection between the first inert electrode (10') and either the steel component (4', 6') or the second inert electrode (10'), and wherein the logical unit (11') imposes the varying signal in the circuit (12').

Sometimes, the volume wherein the electrodes are applied may be a mechanical connection.

Sometimes, the building material may be concrete, grout or asphalt concrete. According to the present specification, the building material may be chosen from the list consisting of concrete and grout.

### BRIEF DESCRIPTION OF THE DRAWINGS

The following description of the figures of specific embodiments of the invention is merely exemplary in nature and is not intended to limit the present teachings, their application or uses. Throughout the drawings, corresponding reference numerals indicate like or corresponding parts and features.
**FIG. 1** is a schematic representation of a section of a part of a wind turbine comprising grout (6) forming a connection between a pile (2) and a transition piece (5).
**FIGs. 2 to 7** present different steps of a method for installing sensors in grout according to the invention.
**FIG. 8** schematically presents a possible application of a method outside the scope of the invention described herein.
**FIG. 9** presents in more detail the part indicated by F2 in FIG 8.
**FIG. 10** presents a cross cut according to line III - III in FIG 9.
**FIGs. 11 and 12** present a different potential application of a method outside the scope of the invention described herein.
**FIGs. 13** **and** **14** present turbine houses (18) in dams (15).

### DETAILED DESCRIPTION OF THE INVENTION

The present invention will be described with respect to particular embodiments but the invention is not limited thereto but only by the claims. Any reference signs in the claims shall not be construed as limiting the scope thereof.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" when referring to recited members, elements or method steps also include embodiments which "consist of" said recited members, elements or method steps.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

The recitation of numerical ranges by endpoints includes all numbers and fractions subsumed within the respective ranges, as well as the recited endpoints.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

One embodiment of the present invention relates to a method for positioning an electrode in grout used in a mechanical connection, the electrode being used for determining the condition of the grout, wherein the method comprises the following steps:
- embedding the electrode in grout such that the electrode is covered with grout, wherein a breakable connection such as wire, string or the like is embedded in the grout together with the electrode, making sure that the breakable connection is at least partially protruding from the grout;
- fixing the grout-encased electrode using the breakable connection to a rod at a desired location;
- positioning the rod with the electrode attached in the desired location in the volume of the mechanical connection where the grout will be applied;
- applying the grout in the mechanical connection by e.g. pouring;
- rupturing or breaking the breakable connection by displacing or moving the rod with respect to the electrode;
- removing the rod from the grout.

An advantage is that such a method allows placing the electrode in the grout of the mechanical connection at a very precise position as the electrode is positioned in the desired location using the rod to which the electrode is connected before the grout is poured. Another advantage is that the electrode is protected during the pouring of the grout through the volume of grout in which it was embedded beforehand.

Moreover, as a result of the method following the invention, only the breakable connection such as the wire or string or the like will remain in the grout. By breaking the breakable connection, the rod can be removed from the ground while the encapsulated electrode remains in the grout. As the grout is very stiff, the encapsulated electrode will not move during the breaking of the breakable connection or when removing the rod. A breakable connection is one that will remain intact during installation and is design to withstand a certain force, and which will break upon the application of a force above a certain limit, for instance, upon rotation or displacement.

By limiting the number of objects remaining in the grout, the influence on the strength of the grout can be kept a minimum.

In some embodiments, the inert electrode may have a mesh form. A mesh is typically a sheet-like material disposed with a regular pattern of apertures. The apertures may be punched into the sheet, or the apertures may form from a crisscross arrangement of wires that form the sheet, for instance, similar to a net. The sheet may be flat or rolled into a structure such as a cylinder.

The apertures may have a maximum width of 0.5mm to 10cm preferably the aperture width is between 0.5 mm and 1% of the smallest dimension (width, height or length) of the grout volume they are applied in.

The mesh can be either a 2D (flat) structure or a 3D (rolled-up sheet or bundle of wires) structure. The thickness of the mesh material (which may comprise sheet or wires) should be below 2.5% of the smallest dimension (width, height or length) of the grout volume it is applied in.

The mesh is preferably connected to an insulated copper conductor which may function for operational coupling with a logic unit. Compared to rod-shaped inert electrodes, similarly sized mesh-shaped inert electrodes generally have a larger surface area. This can result in enhanced measurement sensitivity. Additionally a mesh can be configured to fail (break) at a certain tensile strength; breaking of the mesh corresponding to a serious structural degradation of the grout, can lead to larger signal change

In some embodiments, the mesh is connected to the insulated copper conductor using a spot weld. The copper conductor is electrically insulated (the electrical insulation may be similar to power cable insulation), and after the spot welding process the spot-welded connection as well as the part of the copper cable where the insulation was removed are electrically insulated again using a heat shrink sleeve. The heat shrink sleeve may comprise a thermoplastic filler which is placed over this part of the electrode-cable combination. Preferably, the copper cable only serves to transfer electrical signals and has no mechanical function with relation to the electrode.

Preferably, the rod is positioned such that at least a part of the rod protrudes at least partly above the volume to be filled with grout. The length of the rod is preferably 50 centimeters longer than the depth at which the electrode is positioned in the grout.

This allows removing the rod from the grout by means of the part of the rod which protrudes from the grout. Moreover this will allow keeping the rod at the desired position by attaching the rod to a component of the mechanical connection.

Preferably at least two magnets connected to the rod are used for locating the rod at the desired position. Preferably, the magnets are positioned on a steel part of the mechanical connection. The magnets remain connected to this steel component through magnetic forces. In particular, the magnets are connected to the rod at the protruding part of the rod such that the magnets are not located in the volume of the mechanical connection which will be filled with grout. Alternatively, clamps or other temporary contacting methods may be used instead of magnets. For example, the rod may be clamped to a rebar or the like.

In a practical embodiment one or multiple electrodes are attached to a single rod and/or multiple electrodes are positioned in the volume of the mechanical connection where the grout will be applied.

This has as an advantage that electrodes can be positioned on different locations and on different heights. This will allow controlling the entire volume of the grout using these electrodes.

Aiming to better illustrate the characteristics of the invention, the following is a description of a number of preferred applications of the method for applying an electrode in grout used in a mechanical connection according to the invention. These examples have no limiting character. The description refers to the added figures, wherein:

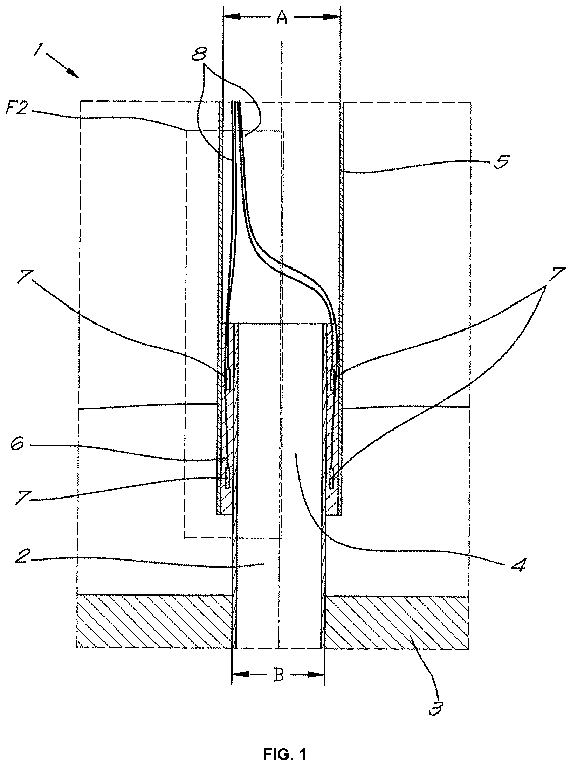

- **FIG. 1** is a schematic representation of a section of a part of a wind turbine;

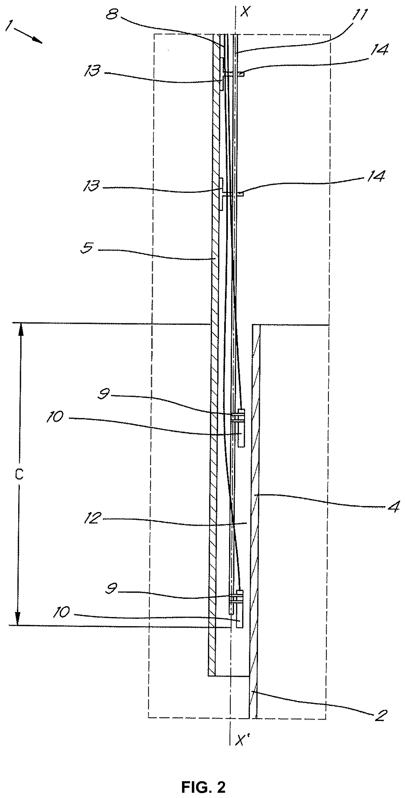

- **FIGs. 2** to **7** schematically present subsequent steps of the method according to the invention.

**FIG. 1** schematically presents a section of a part of a wind turbine 1.

However, it is obvious that the invention is not limited to this application. The method according to the invention can be applied in all sorts of mechanical connections where grout is applied.

In **FIG. 1** the pile 2 is a so-called monopile used to anchor a wind turbine 1 into the seabed is presented, wherein the pile partially protrudes from the seabed 3.

A so-called transition-piece 5 is positioned over this protruding part 4. Alternatively it is also possible that the transition piece 5 is positioned in the pile 2. This transition piece 5 is in turn connected to or is part of the wind turbine 1. The pile 2 and the transition piece 5 are made of steel. The inner diameter A of the transition piece 5 is larger than the outer diameter B of the pile 2. The volume between the pile 2 and the transition piece 5 is filled with grout 6. The grout 6 realizes a mechanical connection between the pile 2 and the transition piece 5, such that the wind turbine 1 is solidly connected to the pile 2 and thus to the seabed 3.

It is also possible to realize the mechanical connection between the pile 2 and the transition piece 5 using bolts or the like in a so-called flanged connection. Grout 6 is applied around the flanged, thereby forming the so-called 'grout skirt'. The 'grout skirt' provides mechanical stabilization, sealing, and corrosion control.

The grout 6 comprises electrodes 7. These electrodes 7 are in this case, though not necessarily, made of a titanium mesh coated with a layer of mixed metal oxide (MMO) or platinum. The mesh is electrically connected to an insulated copper conductor. Compared to rod-shaped electrodes, similarly sized mesh-shaped electrodes generally have a larger surface area. This might result in enhanced measurement sensitivity.

Preferably the apertures of the mesh have a maximum width of 0.5mm to 10 cm, ideally the size is between 0.5 mm and 1% of the smallest dimension (width, height or length) of the grout volume they are applied in.

The electrodes 7 are connected to an electrical cable 8 or the like to enable connecting the electrodes 7 to a logical unit (not present on the figure). The logical unit imposes a current or potential signal between the electrodes 7 or between an electrode 7 and the pile 2 or the transition piece 5.

Based on the resulting signal measured using the logical unit it can be established whether or not mechanical degradation occurred in the grout. The degradation can comprise microcracks, cracks, crevices, gaps, ingress of water, corrosion of the steel, but also detaching or decohesion between the grout 6 and the pile 2 or the transition piece 5.

The electrodes used are relatively vulnerable, making it difficult to apply them in a grout 6 that is typically very tough and viscous before setting. A correct and precise way of positioning the electrodes 7 is very important to obtain good measurement results, to correctly monitor the state of the grout 6. A method described herein will allow realizing this.

**FIGs 2** to **7** present different steps of a method according to the invention.

In a first step the electrode 7 is encapsulated in grout 6 such that the electrode 7 is encased with grout 6. This can be done using a mold or the like wherein the electrode 7 is applied together with a given amount of grout 6.

At the same time a string 9, wire, or the like (i.e. the breakable connection) is partially embedded in the grout 6 as well, such that the wire is at least partially protruding from the grout 6.

Preferably, at least one end of the wire 9 extends for at least five centimeters from the grout 6 of the embedded electrode. In the present case a wire 9 made of nylon is used. In the present case, although not essential, the shape of the embedded electrode is cylindrical. The shape can also depend on the shape of the electrode. It is also possible to embed multiple electrodes 7 together in the grout 6, where the final shape can for example be a longer cylinder compared to the case where only 1 electrode 7 is embedded.

In a next step, the grout-encased embedded electrode 10 is attached to a rod 11 on the desired location using a wire 9. Preferably, the rod 11 has a diameter of about eight millimeter. In the present example, the rod 11 is made of stainless steel.

Because at least one end of the wire 9 protrudes by at least 5 centimeters from the grout 6, attaching the encapsulated electrode 10 to the rod 11 at a desired location may be done easily by wrapping the wire 9 around the rod 11, and subsequently tying the wire 9 in a knot. An alternative approach may employ small holes drilled through the rod (in a direction perpendicular to its X-X' axis) through which the wires can be fed and connected using a knot or alternative method such as a melting joint.

Following this approach, multiple embedded electrodes 10 may be connected to the rod 11, wherein the electrodes 7 are located at different locations along the length of the rod 11. In the example presented in the figures, two embedded electrodes 10 are connected to the rod 11.

Subsequently the rod 11, with attached electrodes 7, is positioned on the desired location in the volume 12 of the mechanical connection wherein the grout will be located.

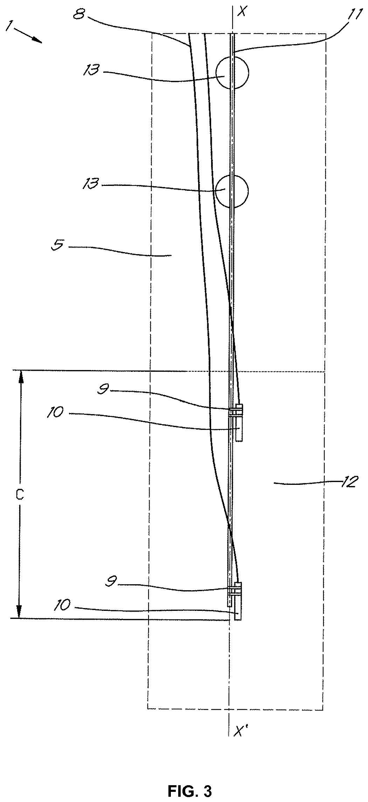

This is presented in **FIGs 2** and **3****.** As can be seen in these figures, the electrical cables 8 of the electrodes 7 are kept pointing upwards such that they may move freely when the grout 6 is poured.

Two magnets 13 are used in this example, wherein the magnets are connected to the rod 11 by means of an L-shaped or elbow- shaped connector.

Using the magnets 13, the rod 11 is fastened to the inner wall of the transition piece 5. This includes taking care that the electrodes 7 are positioned at the desired location and depth.

An alternative method is using one single magnet having two or more connection points. Preferably, the pulling force of the one or more magnet is sufficient to hold the bar in place during pouring of building aggregate such as grout or concrete.

Preferably, the distance between both fixing points (i.e. the distance between two magnets, two points on the same magnet, or one or more clamps having 2 or more fixing points) is selected based on the stiffness and the length of the rod used. Preferably, the amount of fixing points is chosen to limit the lateral displacement of the electrodes connected to the rod during the pouring of building aggregate below a pre-set threshold. The pre-set threshold may be advantageously selected based on the requirements of specific applications.

As can be clearly derived from **FIGs 2** and **3****,** the length of the rod 11 exceeds the depth C at which the bottom electrode 7 is to be positioned in the grout 6. As a result, the rod 11 at least partially protrudes from the volume 12 where the grout will be applied.

The length of rod 11 preferably exceeds this depth C by at least 50 cm. This will allow having both magnets 13 separated by at least 40 cm, preferably 10 centimeters. This will allow preventing a lateral displacement of the rod 11, for example as a result of the forces that can be present during the pouring of the grout.

It is clear that the electrodes 7 can be positioned further or nearer with respect to the inner wall by choosing the appropriate dimensions for the L-shaped connector 14.

It is clear that different rods 11 can be positioned at different locations, for example along the entire inner wall of the transition piece 5 with a regular spacing.

Furthermore, it is clear that the magnets 13 can be fastened to the outer wall of the pile 4 as well.

This principle will allow positioning the desired amount of electrodes 7 very precisely at the desired location. Combining the use of multiple electrodes 7 on a single rod 11, applying multiple rods 11, and using multiple dimensions for the L-shaped connectors 14 will enable locating an electrode 7 at any position in the grout 7.

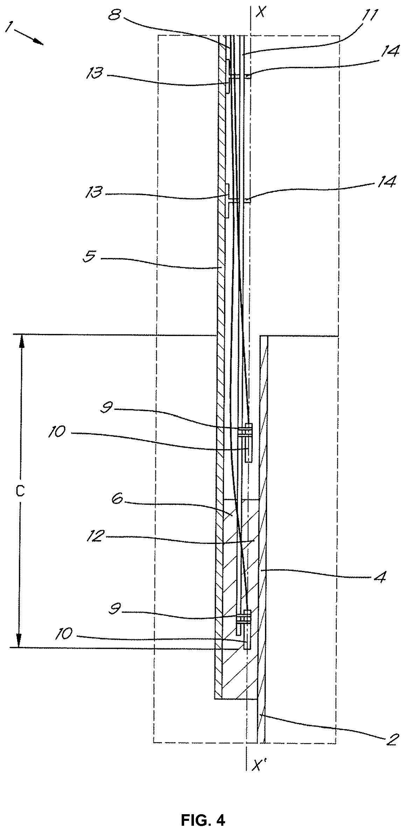

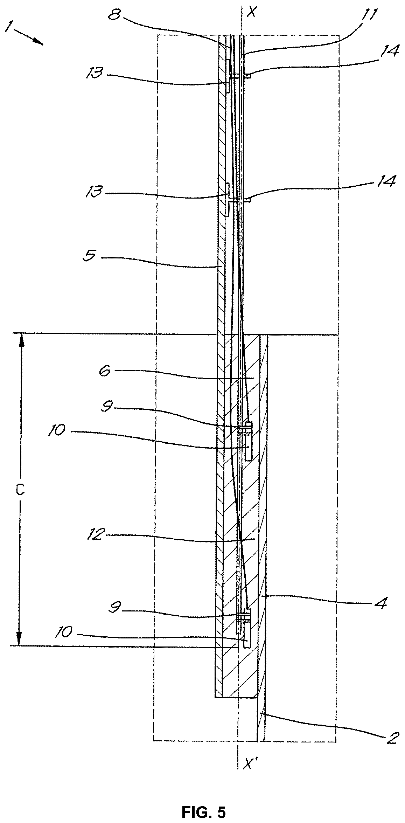

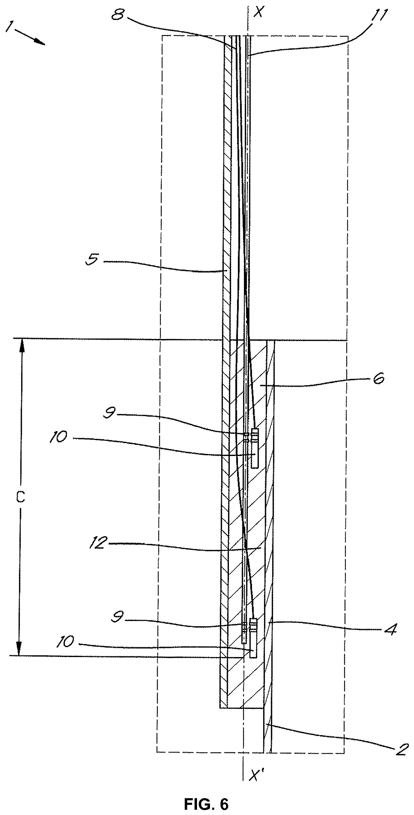

In a next step, the grout 7 is applied by, for example, pouring. This is presented in **FIGs. 4** and **5****.** As can be seen in these figures, the embedded electrodes 10 and the rod 11 will be encased with grout 6. The upper part of the rod 11 and the magnets 13 with the L-shaped connectors 14 are located above the mechanical connection such that they are situated above the grout 6. In a next step the wire 9 is broken by moving or displacing the rod 11 with respect to the embedded electrode 10. This is presented in **FIG. 6** wherein the magnets 13 and the L-shaped connector 14 were already removed.

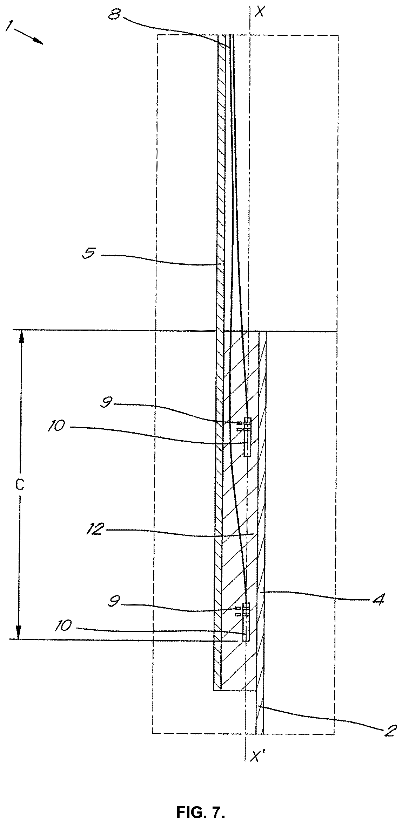

Breaking the wire 9 can be realized by turning the rod 11 around its axis X-X'. As the grout 6 is very stiff and viscous the embedded electrode 10 will not move. In turn the wire 9 will snap. As a weak nylon wire 9 is commonly used this is relatively straightforward. Subsequently the rod 11 can be removed from the grout 6 by pulling it vertically upwards. This is presented in **FIG. 7****.**

These last 2 steps are preferably executed after pouring the grout 6 but before the grout has been set to make sure that the rod 11 can be removed from the grout 6 in a relatively straightforward manner. This enables positioning the electrodes at the desired location very accurately, while making sure that almost no foreign bodies remain in the grout 6. Indeed, apart from the electrode 7, only the breakable connection, such as the wire 9, remains in the grout 6. This has as an advantage that the integrity of the grout 6 is retained to a maximum. The present invention is in no way limited to the given examples or to the embodiments presented in the figures.

In some embodiments, the method may be applied to various building aggregates such as concrete, grout, or asphalt concrete. The aggregates are settable i.e. they harden over time. In particular, the method may be applied to building aggregates that are set, structural or load bearing. In particular, the method may be applied to building aggregates after 21 days of setting. Furthermore, the method may be applied to various constructional structures such as grouted connections, foundations, roads, runways, dams, etc.

Outside the scope of the invention, present specification describes a method for verifying or monitoring the state of grout used in mechanical connections, wherein the method uses a first inert electrode located inside the grout and a steel component that is part of the mechanical connection or is located in its proximity or of a second inert electrode located in the grout, wherein the inert electrodes and the steel component can be connected to each other to generate an electrical circuit, wherein the method comprises the following steps:
- imposing a time-varying current or potential signal in the circuit; and,
- measuring the resulting potential or current signal in the circuit, respectively.

An advantage is that the method outside the scope of the invention allows verification of the state of the grout which can be derived from the resulting signal. Based on the resulting signal, it can be determined if microcracks or cracks are present in the grout, and/or whether a decohesion occurred between the grout and the (steel) wall.

Such a method outside the scope of the invention allows continuous (real time) follow-up or monitoring of the condition of the grout. Moreover the inert electrodes can be easily installed in the grout and they may be cheaper, stronger and more robust compared to optical fibers.

According to the present specification, the inert electrode may have a mesh form. A mesh is typically a sheet-like material disposed with a regular pattern of apertures. The apertures may be punched into the sheet, or the apertures may form from a crisscross arrangement of wires that form the sheet, for instance, similar to a net. The sheet may be flat or rolled into a structure such as a cylinder.

Preferably, the method outside the scope of the invention also comprises the process of determining or recording the amplitude of the measured resulting signal. In addition, the method preferably includes determining or registering the variation in time of the amplitude of the resulting signal. Based on the amplitude and the change over time thereof may allow determining what type of damage or degradation has occurred, and to what extent the damage or degradation has occurred. This may allow determining what steps are required to limit the damage or degradation or to resolve the damage or degradation that occurred.

The method outside the scope of the invention may be adapted for detecting porosity in grout-like materials or building aggregates, such as grout, concrete, mortar, etc... More specifically, the porosity of building aggregates influences their conductivity which may be measured using methods described herein. In dry conditions a higher porosity will result in a higher resistivity, whereas in wet conditions the inverse will be true: higher porosity will result in higher conductivity as the water will fill the pores.

Additionally, wetness and temperature also influence the conductivity of building aggregates. Therefore, methods described hereinmay be applied for monitoring the wetness and/or the temperature of building aggregates

The method outside the scope of the invention may be adapted for determining washing out of grout. Washing out of grout can occur in the context of offshore wind turbines. Referring to Fig 7, the bottom part of the grout will be exposed to the sea water for significant periods of time. Offshore conditions can be very challenging and may include freeze and thaw cycles, tides, and wave activity. As the bottom part of the grouted connection is not always sufficiently sealed off from the outside environment, the bottom side of the grout may interact with those external influences. This may result in a gradual or sudden loss of parts of the grout due to, e.g. progressive cracking or pulverizing. In the case of Fig 7 grout located in the bottom part of the connection will be removed from the connection. As this process progresses, it will reach the location of the bottom electrode 10. This will result in a sudden change in the conductivity measured by this electrode. This allows identifying the 'washing out' of the grout and its progress. Preferably the bottom grout electrode is installed at a level that is lower compared to the critical level of grout coverage such that corrective (re-grouting, adding additional supports...) measures can be taken before the overall stability of the structure affected.

In a practical example, a logical unit is used, wherein the logical unit is electrically connected with the inert electrodes and with the steel component, wherein the logic unit provides the electrical connection between the first inert electrode and either the steel component or the second inert electrode, and wherein the logic unit imposes the time-varying signal in the circuit. In other words, the logic unit can provide in the practical implementation of the method. An advantage of such a logical unit is that it can be set up or tuned on beforehand, i.e. prior to sensor installation in the grout, or from a remote location.

The term "tuned on" as referred herein refers to setting the signal amplitude and/or providing threshold values above or below which an alarm will be issued.

The further treatment of the data, such as storage of the resulting signal, determining the amplitude and the evolution of the amplitude can be performed as well through the logic unit, though this is not essential. It is possible that the logic unit transmits the measured signal to a treatment unit that is not necessarily located in the vicinity of the construction under investigation. Also the interpretation of the data, i.e. determining whether or not damage is present and, if present, where the damage is located based on the amplitude and/or the variation thereof, can be performed inside the logical unit or using a treatment unit.

Preferably, multiple inert electrodes may be located in the grout, each of which can be connected to the steel structure or to another inert electrode to form different circuits; wherein in each of the circuits a varying signal can be applied.

This has as an advantage that the resulting signal in the different circuits can be compared and based on this comparison the orientation, location and progress of the damage such as cracks and microcracks can be determined. This way, not only the presence of damage or degradation can be detected, but also its location. Moreover, this enables determining the evolution of the damage, i.e. the direction in and the rate at which it progresses. This may be achieved through comparing the variation of the amplitude of the different resulting signals.

It is noted that the present method outside the scope of the invention is applicable to load bearing grout. Advantageously it is used not to monitor the setting process, but to monitor structural changes in the grout that arise once it bears a load. It is appreciated in the art that grout can become load bearing after around 21 days of setting. As a general rule, it will achieve around 70% of its strength 21 days after setting. Grout continues to set over time, the setting process lasting months to years, achieving 90 %, or 95 %, or 100 % setting.

The following presents, as an example of the method for examining the state of the grout used in a mechanical connection outside the scope of the invention, wherein:

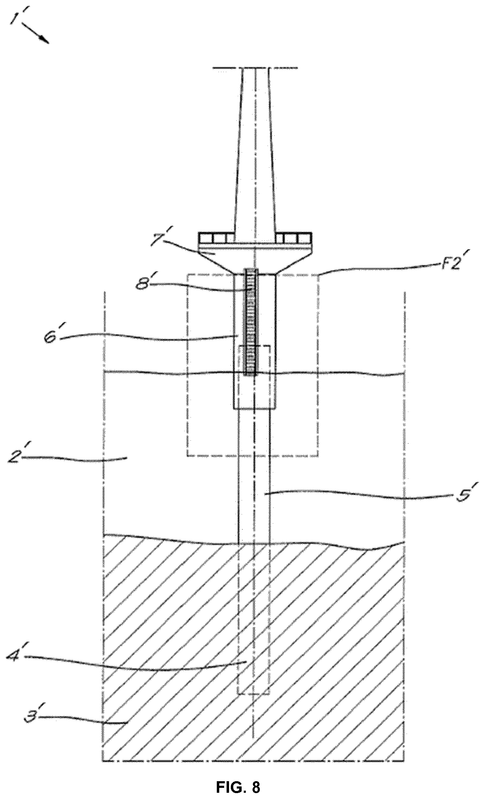

**FIG. 8** schematically presents a possible application of the method described herein;

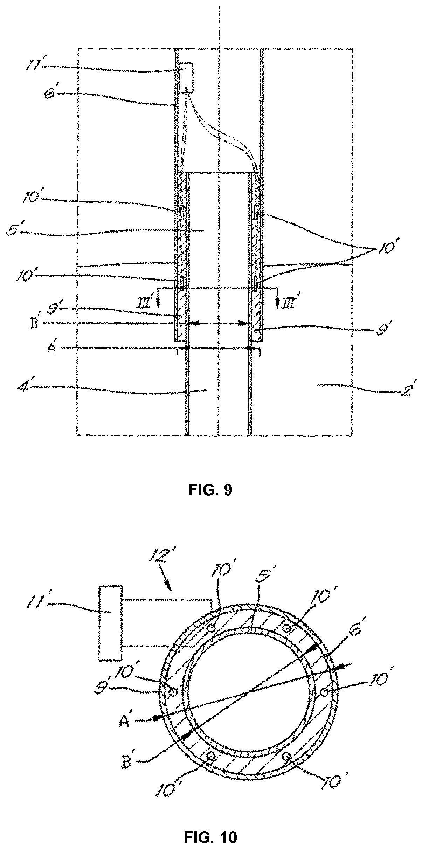

**FIG. 9** presents in more detail the part indicated by F2 in FIG. 8;
**FIG. 10** presents a cross cut according to line III - III in FIG. 9;

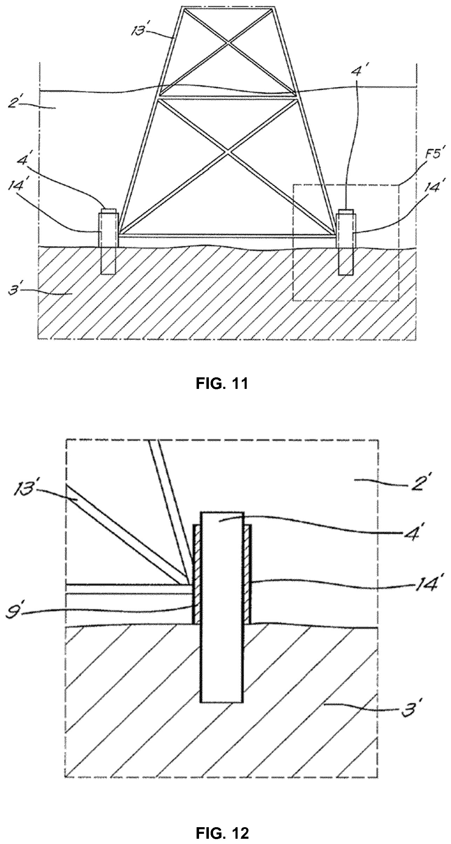

**FIGs. 11** and **12** present a different potential application of the method described herein.

**FIG. 8** shows a schematic representation of the lower part of an offshore wind turbine 1' located in the sea 2'. The wind turbine 1' is connected to the seabed 3' using a pile 4', a so-called 'monopile'. This pile 4' is anchored in the seabed wherein the upper part 5' protrudes above the seabed. A so-called transition piece 6' is positioned over this upper part 5'. The transition piece 6' is connected to or is a part of the wind turbine 1', which contains a working platform 7' that is accessible using a landing stage 8'. The pile 4' and the transition piece 6' are made of steel. The inner diameter A' of the transition piece 6 is larger than the outer diameter B' of the pile 4'.

The space between the pile 4' and the transition piece 6' is filled with grout 9'. This is schematically represented in **FIG. 9****.** The grout 9' will realize a mechanical connection between the pile 4' and the transition piece 6', such that the wind turbine 1' is anchored solidly to the pile 4', and as such at the seabed 3'. It is also possible to realize the mechanical connection between the pile 4' and the transition piece 6' using bolts or similar objects through a so-called flanged connection. Around this flange an amount of grout will be applied, the 'grout skirt'. This 'grout skirt' will provide mechanical stability, but also sealing and corrosion control. In the grout 9' a number of inert electrodes 10' are applied. These are preferably installed during the pouring of the grout 9' in the volume indicated before. These inert electrodes 10' are in this case made of titanium. Moreover these titanium electrodes 10' are coated with a mixed metal oxide. It is also possible that the electrodes are coated with platinum.

As the electrodes 10' are made of an almost inert material it is assumed that the electrochemical potential of the electrodes 10' does not evolve over time resulting from interaction with the surrounding material, in this case the grout 9'. Moreover the electrodes will not degrade through corrosion and such more.

These inert electrodes 10' are in this case uniformly distributed over the volume of the grout 9'. This can be seen in **FIGs. 9** and **10****.** It is also possible that the inert electrodes 10' are concentrated on a specific location in the grout 9', for example where significant amounts of degradation or damage are to be expected. Another possibility is that only one electrode 10' is located in the grout 9', preferably in a location that is sensitive to possible degradation or damage. Such approach can be adopted when a relatively high amount of grout 9' -based connections is to be monitored. All inert electrodes 10' are electrically connected to a logical unit 11', where also the steel transition piece 6' is electrically connected to the logical unit 11'.

Other than the transition piece 6' it is also possible that the pile 4' or another steel component in the vicinity of the grout 9' is electrically connected to the logical unit 11'. The logical unit 11' will have the option to connect electrically the transition piece 6' with any of the inert electrodes 10', resulting in different electrical circuits 12'.

**FIG. 10** shows one circuit 12' in a schematic way using a dashed line. The circuit 12' comprises of a logic unit 11' connected to an electrode 10' and the transition piece 6'. Between the transition piece 6' and the electrode 10' the grout 9' is located, wherein the grout 9' that is located between the transition piece 6' and the electrode 10' is also a part of the circuit 12' or, so to say, closes circuit 10'.

By selecting another inert electrode 10', another circuit 12' can be realized, wherein another part of the grout 9' will be part of this other circuit 12'. In this case the logical unit 11' is capable of imposing a varying current signal in the resulting circuit 12'. As such, the logical unit 11' also contains a current source. Alternatively the logic unit 11' is capable of imposing a varying potential signal. The method outside the scope of the invention to control the state of the grout 9' is very simple and can be summarized as such.

Initially the logical unit 11' electrically connects the transition piece 6' with one of the inert electrodes 10', such that an electrical circuit 12' is generated. The logical unit 11' will impose a varying current signal in the circuit 12'. In other words, the varying current signal is imposed between the electrode 10' and the transition piece 6'. The imposed signal preferably has a sinusoidal shape of which the amplitude is preferably constant. The advantage is that standard alternating current can be used. An alternative method may comprise imposing multi-frequency signals such as so-called multisine signals. Multisine signals comprise a linear combination of sine waves. The amplitudes of the sine waves may vary. Also, the different sine waves may be shifted in phase. Furthermore, the multi-frequency signal may comprise a convolution of multiple sinusoidal signals, or other wave-forms such as square waves, saw tooth waves, or chirped waveforms. Imposing multi-frequency signals has a beneficial effect on the measurement time as well as the information content of the signal response.

The imposed current signal will use the circuit 12', wherein the signal will pass through the grout 9' when it is passing from the transition piece 6' towards the inert electrode 10' or vice versa. Through Ohm's law, the current signal will generate a potential signal in the circuit 12'. Following Ohm's law, the magnitude of the potential is directly proportional to the amplitude of the current. In other words, the amplitude of the measured potential depends on the resistance of the circuit 12'.

This potential signal is measured and is, in this case, recorded in the logical unit 11' such that the data is and will remain available on a later point in time. In this case the logical unit 11' will also determine the amplitude of the recorded signal. The logical unit 11' will also determine the variation over time of the amplitude. Based on these data it can be determine whether or not damage or degradation is incurred by the grout 9'. Indeed, in case microcracks or cracks are present in the grout 9 in the vicinity of the electrode 10', the resistance of the grout 9', and thus the resistance of the circuit 12' will be modified. This will have an impact on the resulting potential signal.

When for example the amplitude of the resulting potential signal slowly increases over a longer period of for example multiple months, this indicates the presence of microcracks in the grout 9'. These microcracks increase the resistance of the grout 9'. When the amplitude decreases this indicates a water uptake of the grout 9' as water uptake will decrease the resistance of the grout. An increased amplitude of the resulting potential signal indicates a possible crack in the grout 9' or a potential decohesion between the pile 4' or the transition piece 6' and the grout. As the amplitude of the varying current signal (imposed) is constant it is relatively simple to identify a decrease of the resulting potential signal.

It is possible to the determine the damage based on an interpretation of the variation in amplitude of the resulting potential signal in the logical unit 11' itself, but alternatively the logical unit 11' can transfer the relevant data to a treatment module that is not necessarily located offshore. The interpretation of the data can be performed in the treatment module itself or through experts or professionals.

The treatment module may be configured for generating an alarm signal or an equivalent when damage, or a too large amount of damage, is observed.

The method outside the scope of the invention described hereinallows monitoring the state of a grout 9 in real time, wherein damage to the grout is identified almost immediately. This has as an advantage that action can be taken well ahead of time, resulting in the necessary steps, e.g. necessary maintenance works.

As multiple electrodes 10' are located in the grout 9', the state of the grout 9' can be verified in multiple locations using the aforementioned principle. Preferably, , the location of the different electrodes 10' may be saved in the logical unit 11'. Preferably, a varying current signal is applied in each of the different circuits 12', and the resulting potential signal recorded. This means that the circuit 12' is broken after measuring the resulting potential signal in the circuit 12' comprising one of the electrodes 10' and the transition piece 6' and that another electrode 10' is connected to the transition piece 6'. A varying current signal is imposed in this new circuit 12' and the resulting potential signal is measured and recorded in the logical unit 11'. The procedure is continued until all electrodes 10' have been covered, after which the procedure can be repeated.

Preferably, the same signal may be imposed in every circuit 12'. This has as an advantage that the logical unit 11' requires only 1 type of current source and that the interpretation of the measured data is more straightforward. Following this principle it is possible to verify the state of the grout 9' near the location of the electrodes 10'.

By comparing the resulting signal of the different circuits 12' it is possible to identify the orientation and progress of any damage as the location of the electrodes 10' is retained in the logical unit 11'. As such, the damage as well as its location and progress can be determined.

Although the example presented above always mentions a circuit 12' generated between one electrode 10' and the steel transition piece 6', it cannot be excluded that the circuit 12' is realized between 2' of the inert electrodes 10'. In that case, the logical unit 11' will realize, for example, an electrical connection between two electrodes 10' to generate an electrical circuit 12'. This can be the case when for example the grout 9' realizes a mechanical connection between parts that are not made of an electrically conductive material such as steel.

**FIGs. 11** and **12** present an alternative application of the method described herein. The specification describes an offshore construction where a so-called jacket foundation 13' is anchored to the seabed using 4 legs 14' that can be positioned over 4 piles 4'.

As can be seen in **FIG. 12****,** the void between the piles 4' and the legs 14' is filled with grout 9' to realize a strong mechanical connection.

Inert electrodes 10' are subsequently embedded in the grout 9', whereby the electrodes are used for monitoring the state of the grout 9', similarly as in the example described above.

In this case, the state of the grout 9' is controlled for four mechanical connections. This can be done using one logical unit 11', or using 4 logical units 11', one for each pile 4'.

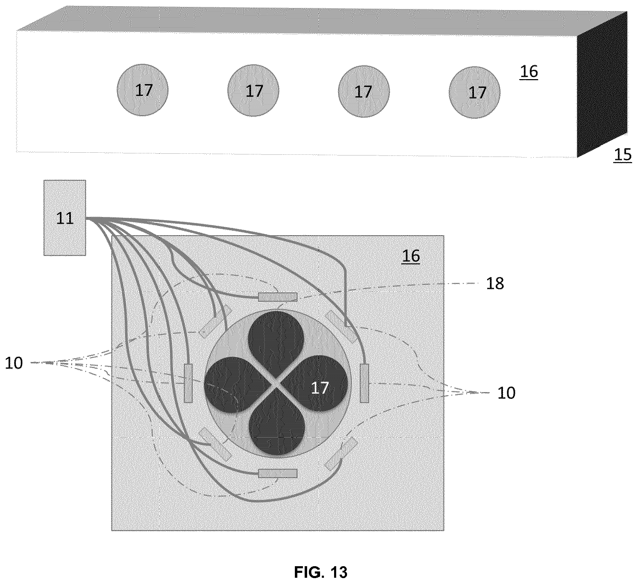

An alternative application is described in **FIGs. 13** to **14****.** The application illustrates water-powered generators embedded in a concrete base structure such as hydropower dams or dams 15 used to harvest tidal energy. These dams comprise a concrete base structure in which one or more turbines are housed. Such a turbine is located in a so-called turbine house 18 which is made of steel. The turbine 17 rotates for a large part of its operational life. As a result, the turbine house 18 it is located in exerts dynamic forces on the surrounding material which is commonly a building aggregate such as grout or concrete. These dynamic forces, together with the constant presence of water and/or moisture, may result in a degradation of the surrounding material. This may result in a reduced stability of the turbine 17 in the surrounding material 16. As a result the performance of the turbine 17, thus the electricity production can be negatively influenced or repairs may become required. This can be prevented by e.g. modifying the operational parameters and/or by injecting the surrounding material 16 with an additional grout once cracks or other damage is detected.

This detection can be performed using the method disclosed herein. This may be achieved by distributing a set of electrodes 10 around the steel support structure 18 of the turbine 17. The electrodes 10 are positioned in the volume prior to grouting or casting concrete. Though not essential, the electrodes 10 may be distributed uniformly around the circumference of the turbine house 18, as shown in **FIG. 13****.**

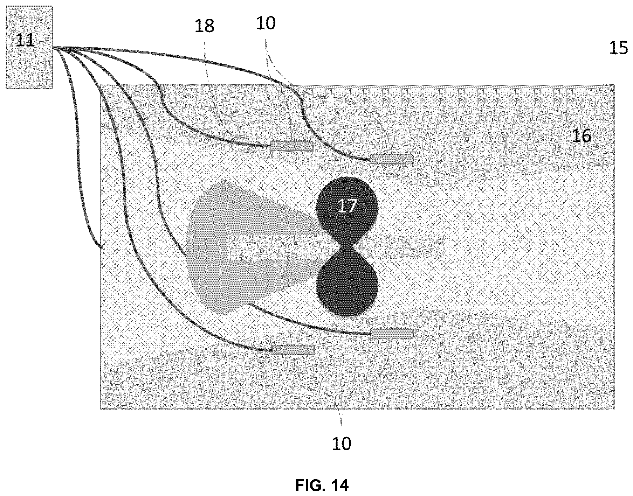

For example, two sets of electrodes are installed surrounding the turbine house 18 in order to capture with sufficient detail the location and potential progress of the damage. This is shown in **FIG. 14****.** The electrodes 10 as well as the steel turbine house 18 are connected to the logical unit 11. After the building aggregate has set, and the sensors are operational, an AC current signal is applied between the inert electrodes 10 and the turbine house 18. The resulting AC potential response is recorded and analyzed. This allows detecting cracks in the grout or concrete as well as decohesion between grout or concrete and steel. Also water uptake or microcracking can be detected. The same type of signal can be applied as well between 2 neighboring electrodes 10. Again the response signal is captured and treated. Based on the conductivity measured, which is in itself derived from the applied current and the measured voltage (or vice versa), one or more diagnosis can be made chosen from the list comprising: determining whether or not cracks are present in the volume between both electrodes, determining whether or not microcracking is taking place in the same volume, and determining whether or not the water content is changing (e.g through moisture ingress into the building aggregate). Evidently, the specific amounts of electrodes and/or sets of electrodes listed herein are not limiting; more or less electrodes may be used, based on the particular design of constructions in the context of which electrodes as described herein are applied.

It is clear that the method for applying an electrode in a building material that is settable according to the invention, and its applications, are not limited to the presented examples.

In particular, methods according to the invention may be applied in mechanical connections that are not located in offshore constructions.

Indeed, the method may be applied to virtually any mechanical connection which may be realized using grout 9'.

In methods compatible with the present invention, damage may be located by comparing the response of sensors in different positions and/or by looking at the evolution of the response of the different individual sensors in time. If the response (e.g. the amplitude of a current or voltage signal) detected by a single sensor starts deviating by more than a certain predetermined value, for example two percent compared to the values obtained in a previous period, and the response of the other sensors located in the same structure or component does not change by more than a certain predetermined value, for example two percent, then the damage is located in the vicinity of this single sensor. If now in a later stage, for example 3 days later, the amplitude response of a neighboring sensor starts deviating as well by an amount larger than a certain predetermined value, for example more than two percent compared to the values recorded earlier, then the damaged zone has spread and is now covering a larger part of the volume of the structure or the component. Evidently, this procedure may be applied *mutatis mutandis* to further sensors located in the building aggregate.

In some embodiments, the method may be applied to various building aggregates such as concrete, grout, or asphalt concrete. The aggregates are settable i.e. they harden over time. In particular, the method may be applied to building aggregates that are set, structural or load bearing. In particular, the method may be applied to building aggregates after 21 days of setting. Furthermore, the method may be applied to various constructional structures such as grouted connections, foundations, roads, runways, dams, etc.

It is noted that the monitoring method outside the scope of the invention described herein may be applied to electrodes installed using the installation method according to the invention described herein.

## Claims

1. Method for positioning an electrode (7) in a building material (6) that is settable, wherein the electrode (7) is used for determining the condition of the building material (6), comprising the following steps:
- embedding the electrode (7) in the building material (6) such that the electrode (7) is encased with the building material (6), thereby forming an encased electrode (10), wherein a breakable connection (9) is additionally partially embedded into said building material (6) such that the breakable connection at least partially protrudes from the casing of the encased electrode said breakable connection being a wire or string or the like which will remain intact during installation and which is designed to break upon the application of a force above a certain limit;
- connecting the encased electrode (10) using the breakable connection to a rod (11);
- positioning the rod (11) with the encased electrode (10) in a desired location in a volume (12) where the building material (6) will be applied;
- applying the building material (6) in the volume (12), for example through pouring;
- breaking or rupturing the breakable connection by moving or displacing the rod (11) with respect to the electrode (7);
- removing the rod (11) from the building material (6).

2. Method according to claim 1 wherein the rod (11) is positioned to protrude at least partially above the aforementioned volume when positioning the rod (11), wherein, preferably, the length of the rod (11) exceeds the depth (C) at which the electrode (7) is to be positioned in the building material (6) by at least fifty centimeters.

3. Method according to claim 1 or 2 wherein, for applying the rod (11) at a desired location, at least two magnets (13) are used, wherein the magnets (13) are attached to the rod (11), and wherein the magnets (13) are positioned on a steel part (2, 5), for example a steel part of a mechanical connection.

4. Method according to claim 3 wherein both magnets (13) are separated from each other by at least 10 centimeters.

5. Method according to any one of the previous claims wherein the breakable connection is broken or ruptured by rotating the rod (11).

6. Method according to any one of the previous claims wherein multiple electrodes (7) are attached to a single rod (11).

7. Method according to claim 6 wherein during the process of encasing the electrodes (7) with the building material, multiple electrodes are encased in the building material, thereby forming encased electrodes.

8. Method according to any of claims 1 to 7 wherein the building material is concrete, grout or asphalt concrete.

## Patentansprüche

1. Verfahren zur Positionierung einer Elektrode (7) in einem Baumaterial (6), das abbindbar ist, wobei die Elektrode (7) zum Bestimmen des Zustands des Baumaterials (6) verwendet wird, die folgenden Schritte umfassend:
- Einbetten der Elektrode (7) in das Baumaterial (6), sodass die Elektrode (7) mit dem Baumaterial (6) umschlossen ist, dabei eine umschlossene Elektrode (10) bildend, wobei eine brechbare Verbindung (9) zusätzlich teilweise in das Baumaterial (6) eingebettet ist, sodass die brechbare Verbindung zumindest teilweise aus der Umschließung der umschlossenen Elektrode herausragt, wobei die brechbare Verbindung ein Draht oder eine Schnur oder ähnliches ist, die während der Installation intakt bleibt und die so konzipiert ist, dass sie bei Anwendung einer Kraft über einer bestimmten Grenze bricht;
- Verbinden der umschlossenen Elektrode (10) unter Verwendung der brechbaren Verbindung mit einer Stange (11);
- Positionieren der Stange (11) mit der umschlossenen Elektrode (10) in einer gewünschten Position in einem Volumen (12), wo das Baumaterial (6) eingebracht wird;
- Einbringen des Baumaterials (6) im Volumen (12), beispielsweise durch Gießen;
- Brechen oder Reißen der brechbaren Verbindung durch Bewegen oder Versetzen der Stange (11) bezüglich der Elektrode (7);
- Entfernen der Stange (11) aus dem Baumaterial (6).

2. Verfahren nach Anspruch 1, wobei die Stange (11) positioniert wird, um zumindest teilweise über dem erwähnten Volumen herauszuragen, wenn die Stange (11) positioniert wird, wobei, vorzugsweise, die Länge der Stange (11) die Tiefe (C), bei der die Elektrode (7) im Baumaterial (6) zu positionieren ist, um mindestens fünfzig Zentimeter übersteigt.

3. Verfahren nach Anspruch 1 oder 2, wobei, zum Anwenden der Stange (11) an einer gewünschten Position, mindestens zwei Magnete (13) verwendet werden, wobei die Magnete (13) an der Stange (11) befestigt sind und wobei die Magnete (13) an einem Stahlteil (2, 5), beispielsweise einem Stahlteil einer mechanischen Verbindung, positioniert sind.

4. Verfahren nach Anspruch 3, wobei beide Magnete (13) voneinander mindestens 10 Zentimeter getrennt sind.

5. Verfahren nach einem der vorhergehenden Ansprüche, wobei die brechbare Verbindung durch Drehen der Stange (11) gebrochen oder zerrissen wird.

6. Verfahren nach einem der vorhergehenden Ansprüche, wobei mehrere Elektroden (7) an einer einzelnen Stange (11) befestigt sind.

7. Verfahren nach Anspruch 6, wobei während des Prozesses des Umschließens der Elektroden (7) mit dem Baumaterial mehrere Elektroden in das Baumaterial eingeschlossen werden, dabei umschlossene Elektroden bildend.

8. Verfahren nach einem der Ansprüche 1 bis 7, wobei das Baumaterial Beton, Mörtel oder Asphaltbeton ist.

## Revendications

1. Procédé pour positionner une électrode (7) dans un matériau de construction (6) qui est capable de prise, dans lequel l'électrode (7) est utilisée pour déterminer la condition du matériau de construction (6), comprenant les étapes suivantes:
- l'encastrement de l'électrode (7) dans le matériau de construction (6) de telle sorte que l'électrode (7) soit enrobée avec le matériau de construction (6), ainsi formant une électrode enrobée (10), dans lequel une connexion cassable (9) est de plus partiellement encastrée dans ledit matériau de construction (6) de telle sorte que la connexion cassable fasse saillie au moins partiellement à partir de l'enrobage de l'électrode enrobée, ladite connexion cassable étant un fil ou un câble ou analogue qui restera intact durant l'installation et qui est conçu pour se casser lors de l'application d'une force supérieure à une certain limite ;
- la connexion de l'électrode enrobée (10), en utilisant la connexion cassable, à une tige (11) ;
- le positionnement de la tige (11) avec l'électrode enrobée (10) dans un emplacement souhaité dans un volume (12) où le matériau de construction (6) sera appliqué ;
- l'application du matériau de construction (6) dans le volume (12), par exemple par l'intermédiaire de coulage ;
- le cassage ou la rupture de la connexion cassable en mouvant ou déplaçant la tige (11) par rapport à l'électrode (7) ;
- l'enlèvement de la tige (11) à partir du matériau de construction (6).

2. Procédé selon la revendication 1 dans lequel la tige (11) est positionnée pour faire saillie au moins partiellement au-dessus du volume susmentionné lors du positionnement de la tige (11), dans lequel, de préférence, la longueur de la tige (11) dépasse la profondeur (C), à laquelle l'électrode (7) est destinée à être positionnée dans le matériau de construction (6), d'au moins cinquante centimètres.

3. Procédé selon la revendication 1 ou 2 dans lequel, pour l'application de la tige (11) à un emplacement souhaité, au moins deux aimants (13) sont utilisés, dans lequel les aimants (13) sont attachés à la tige (11), et dans lequel les aimants (13) sont positionnés sur une pièce en acier (2, 5), par exemple une pièce en acier d'une connexion mécanique.

4. Procédé selon la revendication 3, dans lequel les deux aimants (13) sont séparés l'un de l'autre d'au moins 10 centimètres.

5. Procédé selon l'une quelconque des revendications précédentes, dans lequel la connexion cassable est cassée ou rompue en tournant la tige (11).

6. Procédé selon l'une quelconque des revendications précédentes, dans lequel de multiples électrodes (7) sont attachées à une seule tige (11).

7. Procédé selon la revendication 6, dans lequel, au cours de l'enrobage des électrodes (7) avec le matériau de construction, de multiples électrodes sont enrobées dans le matériau de construction, ainsi formant des électrodes enrobées.

8. Procédé selon l'une quelconque des revendications 1 à 7, dans lequel le matériau de construction est du béton, un coulis ou du béton asphaltique.
